# EUROPEAN PATENT APPLICATION

(11) **EP 4 190 899 A1**
(43) Date of publication of application: **07.06.2023**
(21) Application number: 21848698.3
(22) Date of filing: 04.06.2021
(51) Int. Cl.: C12N 15/113, C12Q 1/68

(54) **MCI DIAGNOSTIC MARKER, MCI DIAGNOSTIC KIT, AND CORRESPONDING DETECTION METHOD**

(30) Priority: 31.07.2020 CN 202010760263
(71) Applicant: Shanghai Mental Health Center (Shanghai Psychological Counselling Training Center), Shanghai 200030 (CN)
(72) Inventor: WANG, Tao, Shanghai 200030 (CN); ZHANG, Wei, Shanghai 200030 (CN); XIAO, Shifu, Shanghai 200030 (CN)
(74) Representative: dompatent von Kreisler Selting Werner - Partnerschaft von Patent- und Rechtsanwälten mbB
(86) International application number: PCT/CN2021/098317
(87) International publication number: WO 2022/022060

(57) **Abstract**

Disclosed a diagnostic marker for prodromal stage Mild Cognitive Impairment (MCI) of the Alzheimer's disease. The marker is plasma long-chain non-coding ribonucleic acid (lncRNA), comprising one or more of ENST00000549762, NR_024049, T324988 or ENST00000567919. Also disclosed are an application of the marker and a corresponding kit.

## Description

### INCORPORATION BY REFERENCE TO ANY PRIORITY APPLICATIONS

This application claims priority of the Chinese invention patent application with application number 202010760263.8 filed on July 31, 2020, the contents of which are hereby incorporated by reference.

### BACKGROUND OF THE INVENTION

### Field of the Invention

The present invention relates to the prodromal phase of Alzheimer's disease, in particular to diagnostic markers, and specifically refers to a diagnostic marker of Mild Cognitive Impairment and application thereof.

### Description of the Related Art

Alzheimer's disease (AD) is a chronic neurodegenerative disease, which has become the most common type of senile dementia. Its clinical manifestations are the deterioration of cognitive and memory functions, progressive decline of daily living ability, and various neuropsychiatric symptoms and behavioral abnormalities. The main pathological features were senile plaques formed by the deposition of extracellular amyloid protein and apoptosis of cerebral cortical neurons caused by neurofibrillar tangles formed by the hyperphosphorylation of microtubule related Tau protein in nerve cells. With the aging of the population, the incidence rate of Alzheimer's disease is increasing year by year. According to the world annual report on Alzheimer's disease, there is a new dementia patient every three seconds, and the number of patients has reached 50 million. It is estimated that this number will reach 152 million by 2050. At present, the incidence of Alzheimer's disease in China has reached more than 8 million. There is no effective treatment to prevent or reverse the disease, and only drugs can improve its symptoms. It has brought a heavy burden to medical care and nursing. For example, the average annual medical care cost of each intermediate and advanced AD patient in the United States is up to 50000 dollars. With the acceleration of aging in China, the medical care and economic burden caused by AD will become more and more serious. Therefore, it is necessary to further explore the pathogenesis of AD, early diagnosis and early intervention of disease development, and find effective treatment methods to reduce the burden of medical care. This is a medical, health and socio-economic issue that must be solved in an aging society.

Mild cognitive impairment (MCI) is a transitional state of cognitive function between normal aging and dementia. MCI can be divided into two subtypes: forgetting type MCI and non-forgetting type MCI. Forgetting type MCI is increasingly considered as the precursor of AD. Research shows that about 10-15% of patients with Forgetting type MCI progress to possible AD every year. MCI is a high-risk group that needs intervention, and is also the best entry point for early prevention and intervention. In the past 20 years, the diagnosis of AD and MCI has made great progress. At present, the diagnosis method of AD and MCI is mainly through the consultation of professional doctors, who judge according to the medical history and scale scores, mainly including the Montreal Cognitive Assessment Scale, complete neuropsychological test, simple mental state scale and clinical dementia scale, but the evaluation of the scale is time-consuming, labor-intensive, and relatively inaccurate. The auxiliary examination mainly includes the imaging examination of brain structure, which can observe the macroscopic changes of the patient's brain structure, but cannot find the characteristic changes of MCI in the early stage. As the current gold standard for AD diagnosis, senile plaque imaging can see pathological changes, but it is expensive and has no clinical application in China. Therefore, there is no clinical biomarker early diagnosis method that can be popularized and applied clinically in China. Exploring the use of peripheral blood biomarkers to screen potential MCI patients will help the early diagnosis and treatment of AD.

Long non-coding RNA (lncRNA) is a class of RNA molecules with a length of more than 200 nucleotides. It is usually a by-product of RNA polymerase II transcription and cannot encode protein. It was once considered as "noise" of transcription. Recent studies have found that lncRNA is specific in different human tissues and plays a variety of biological functions, especially in the development of the nervous system, neuronal differentiation, synaptic plasticity and other processes. It has been proved that the differential expression of lncRNA in the brain of Alzheimer's disease patients, and the abnormal regulation of lncRNA regulates the production of Aβ during the development of Alzheimer's disease. It plays an important role in synaptic damage, consumption of neurotrophic factors, inflammatory response, mitochondrial damage and neuronal stress response, and affects the occurrence and development of Alzheimer's disease. LncRNA is not only abundant in brain tissue, but also stable in cerebrospinal fluid, plasma and serum samples. Among human biological samples, peripheral blood samples are the easiest to obtain, with simple operation, minimal trauma, and the least risk and pain borne by patients. Blood is considered to be the most suitable biological sample for screening high-risk groups of AD and for early detection, diagnosis and treatment intervention follow-up of AD. The structural characteristics of LncRNA endow it with the ability to be detected and become a stable plasma biomarker for MCI and AD diagnosis without the influence of endogenous RNase activity. The results of lncRNA study provided by the applicant suggest that the expression of several genetic markers lncRNA in peripheral blood is different between MCI and normal control. The detection of the expression level of plasma lncRNA can be used as a routine means for screening early diagnosis of AD, and optimize the clinical diagnosis strategy for AD.

### SUMMARY OF THE INVENTION

The purpose of the present invention is to overcome the above-mentioned shortcomings of the prior art, and provide diagnostic marker of mild cognitive impairment and application therefore that can effectively screen and diagnose AD and optimize clinical AD diagnosis strategies.

In order to achieve the above objective, one aspect of the present invention provides a diagnostic marker of mild cognitive impairment (MCI), wherein the marker is plasma lncRNA comprising one or more of ENST000000549762, NR_024049, T324988 or ENST000000567919.

The present invention also provides an application of the diagnostic marker of mild cognitive impairment in the preparation of a diagnostic kit for mild cognitive impairment.

The present invention also provides a diagnostic kit for mild cognitive impairment, wherein the kit for measuring the content of one or more of ENST000000549762, NR_024049, T324988 or ENST000000567919 in plasma.

Preferably, the kit comprising primers and probes of one or more of ENST000000549762, NR_024049, T324988 or ENST000000567919.

Preferably, the kit comprising the internal control 18S.

Preferably, the formula for calculating the content of one or more of ENST000000549762, NR_024049, T324988 or ENST000000567919 in plasma measured by the kit is:
(1) -4.749+7972.194×ENST00000549762+5609.072×NR_024049+5.073×T324988+961.747 ×ENST00000567919;
(2) -4.628+8552.604×ENST00000549762+5885.819×NR_024049+6.017×T324988;
(3) -4.549+9376.777×ENST00000549762+6908.534×NR_024049+2195.991 ×ENST00000567919;
(4)-4.220+6823.570×NR_024049+5.849×T324988+1213.731×ENST00000567919;
(5) -4.129+6.122×T324988+1128.888×ENST00000567919+9678.308×ENST00000549762;
(6) ENST00000549762;
(7) NR_024049;
(8) T324988; or,
(9) ENST00000567919.

A method of using the diagnostic kit for mild cognitive impairment, wherein comprising:
(1) Using a lncRNA reverse transcription kit to reverse transcript the total RNA to be detected to obtain corresponding cDNA;
(2) performing real-time fluorescence quantitative PCR with the cDNA, and using 18S as an internal control, detection result is expressed as ΔCt, wherein ΔCt= Ct lncRNA - Ct 18S; and
(3) processing the result ΔCt according to a formula:
   -4.749+7972.194×ENST00000549762+5609.072×NR_024049+5.073×T324988+961.747 ×ENST00000567919, and comparing a calculated value with -0.8392;
   -4.628+8552.604×ENST00000549762+5885.819×NR_024049+6.017×T324988, and comparing a calculated value with -0.6984;
   -4.549+9376.777×ENST00000549762+6908.534×NR_024049+2195.991 ×ENST000000567919, and comparing a calculated value with -0.5859;
   -4.220+6823.570×NR_024049+5.849×T324988+1213.731×ENST000000567919, and comparing a calculated value with -0.3367;
   -4.129+6.122×T324988+1128.888×ENST00000567919+9678.308×ENST000000549762, and comparing a calculated value with -0.2550;
   ENST000000549762, and comparing a calculated value with 0.000160542039;
   NR_024049, and comparing a calculated value with 0.000235892192;
   T324988, and comparing a calculated value with 0.243255710000; or,
   ENST000000567919, and comparing a calculated value with 0.000502313314.

The beneficial effects are: through rigorous experiments and statistical analysis, the biomarkers ENST000000549762, NR_024049, T324988 and ENST000000567919 with high diagnostic value for amnestic mild cognitive impairment are disclosed. Through the development and application of lncRNA markers and diagnostic kits, we have broken through the dilemma of lacking convenient peripheral plasma diagnostic markers of amnestic mild cognitive impairment, which is conducive to the early diagnosis and early intervention of Alzheimer's disease dementia. At the same time, it is hopeful to greatly promote the discovery of new anti-AD therapeutic drug targets with potential therapeutic value and provide scientific basis and clinical support.

### BRIEF DESCRIPTION OF THE DRAWINGS

Figure 1 is a flow chart of the lncRNA combinatorial chip screening, training and verification experiment design for identifying target plasma of MCI patients according to the present invention.
Figure 2 is a diagram of the main method steps for determining the plasma lncRNA combination of MCI patients in the diagnosis of the present invention.
Figure 3 shows the ROC curve of MCI patients diagnosed with 4 lncRNA combinations.
Figure 4 shows the ROC curve of MCI patients diagnosed with 3 lncRNA combinations.
Figure 5 shows the ROC curve of MCI patients diagnosed with an lncRNA.

### DETAILED DESCRIPTION OF THE PREFERRED EMBODIMENT

In order to be able to describe the technical content of the present invention more clearly, further description will be given below in conjunction with specific embodiments.

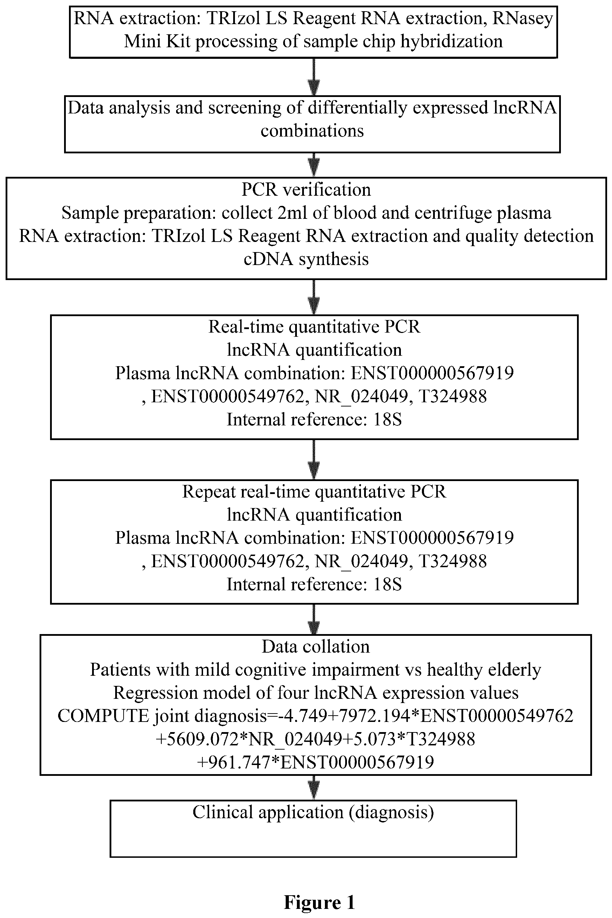

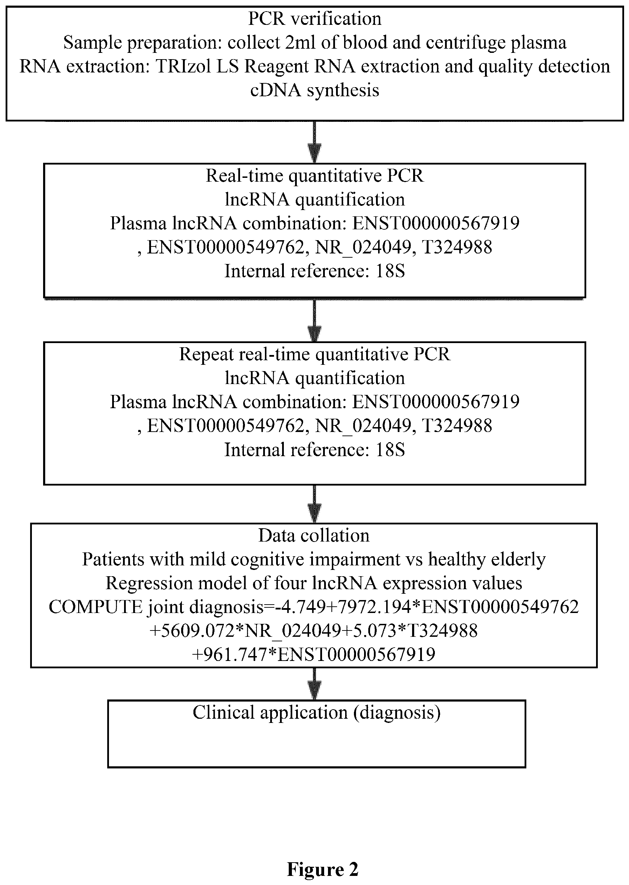

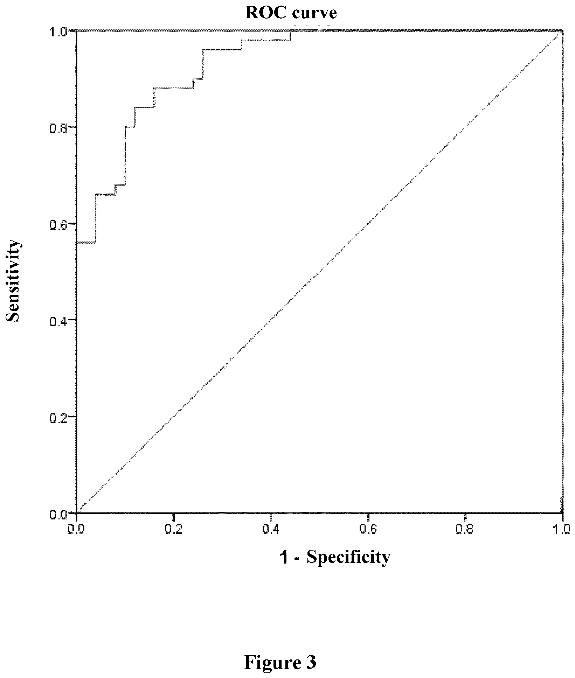

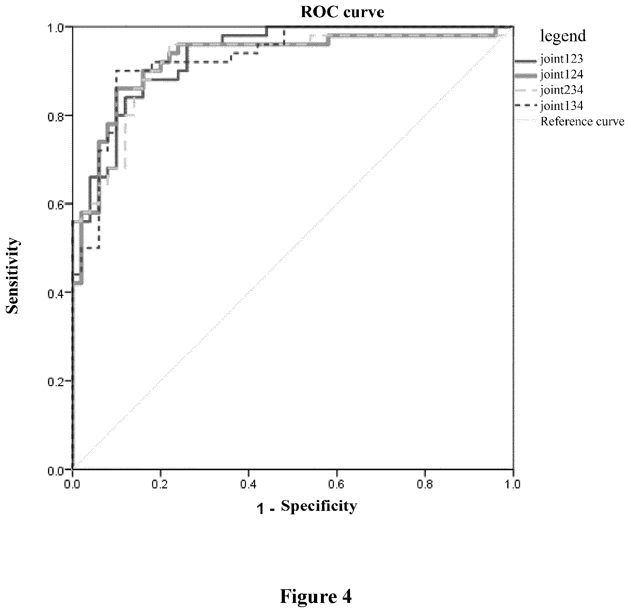

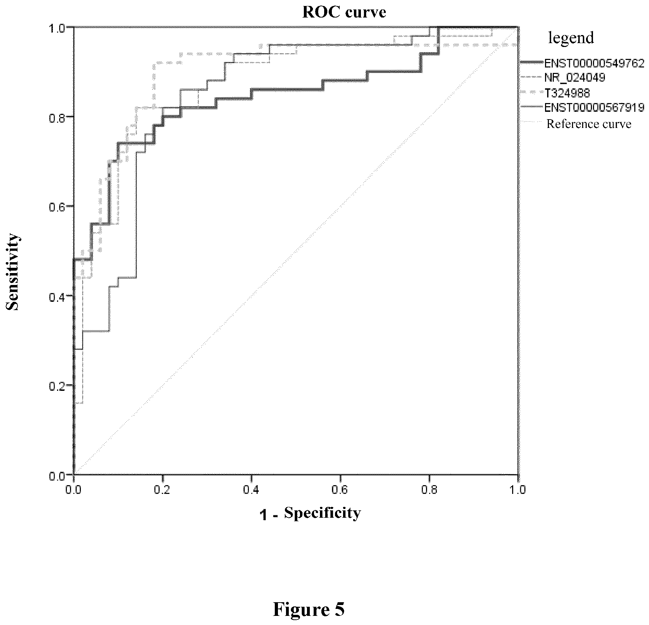

With reference to Figures 1 to 5, the screening and verification of diagnostic marker for MCI according to the present invention will be specifically described.

### I. Research object

The subjects were 50 elderly cases of MCI collected in the communities of XuHui District, Shanghai. The control group was healthy elderly people with matching age, sex, and years of education.

### II. Research method

### 1. Chip screening

(1) Extract RNA using TRIzol method and purify it with RNasey Mini Kit (GIAGEN). Use Agilent ND-1000 to measure the purified RNA concentration, and electrophoresis to detect RNA integrity.
(2) After the extracted RNA passes the quality inspection, use the Arraystar RNA Flash Labeling Kit to label the lncRNA. After the labeling is completed, hybridize the sample with Array human lncRNA chip(v4.0) using Agilent SureHyb, and the total reaction volume is 50µl.
(3) After washing, the chip was scanned with Agilent DNA Microarray Scanner. Use Agilent Feature Extraction software to collect chip signal values. Use Agilent GeneSpring GX v12.1 software to standardize the chip and screen the differentially expressed lncRNAs.

### 2. Real-time quantitative PCR verification

After chip screening, select lncRNAs that are significantly down-regulated compared with the control group for real-time quantitative PCR verification. The specific implementation methods are as follows:
(1) Extract RNA using TRIzol method and purify it with RNasey Mini Kit (GIAGEN). Use NanoDrop ND-1000 to measure the purified RNA concentration.
The extracted RNA undergoes a reverse transcription reaction after quality inspection. The total reaction volume is 20ul (total RNA 300ng, reverse transcription specific primer 1µl, dNTP1.6 µl, water without nuclease added to 13.5 µl, RNA inhibitor 0.5 µl, reverse transcriptase 1 µl, buffer 4 µl, 0.1 M DTT1 µl), and the reaction is carried out for different duration (60 minutes, 15 minutes) at different temperatures (50 °C, 70 °C).
(2) The total system of real-time quantitative PCR amplification was 10 µl, which was carried out at 95 °C for 10 minutes, and then reacted for 40 PCR cycles (95 °C, 10 seconds; 60 °C, 60 seconds). Taking 18S as the internal control, the test result is expressed in 2-ΔΔCt relative to the amount of the reference substance. The smaller the 2-ΔΔCt, the lower the expression amount. The primer information used by PCR is shown in Table 1.

**Table 1: primer information of Real-time quantitative PCR**

| Gene name | Bidirectional primer sequence | annealing temperature (°C) | Product length (bp) |
|---|---|---|---|
| 18S rRNA | F : 5'CAGCCACCCGAGATTGAGCA3' | 60 | 252 |
| | R : 5'TAGTAGCGACGGGCGGTGTG3' | | |
| NR_024049 | F:5'GATAAAATGCCACACAACCAT 3' | 60 | 77 |
| | R:5' CAGAACTCCATAGCCACCAA 3' | | |
| ENST00000549762 | F:5'GGTCACATCAGGGCACATT 3' | 60 | 70 |
| | R:5'TGACTGCTGAGAAAGGGACA 3' | | |
| NR_040772 | F:5'GACCCACGTGAAGCGTACAG 3' | 60 | 56 |
| | R:5'CTCTTCCGCACCGTCCTTC 3' | | |
| T324988 | F:5' GAGATGGGGTTTTGTCATGTTAG3' | 60 | 55 |
| | R: 5'TACCTGAGGTCAGGAGATCGAG3' | | |
| ENST00000567919 | F:5'GTTTCAGGTGCAGGCGTGT 3' | 60 | 96 |
| | R:5'CCCTCACTTCCTATGCCAATC 3' | | |

### III. Research results

During chip screening stage, the expression levels of ENST00000567919, T264003, T286616, T324988, ENST00000549762, NR_024049, NR_040772 in the MCI group are significantly lower than the normal control group. The specific data are shown in Table 3 below:

**Table 3: Differential levels of lncRNA expression in the normal control group (NC) and mild cognitive impairment (MCI) groups**

| LncRNA | Fold Change | P Value | FDR |
|---|---|---|---|
| ENST00000567919 | 80.994 | 0.009 | 0.859 |
| T264003 | 13.274 | 0.001 | 0.688 |
| T286616 | 22.967 | 0.020 | 0.859 |
| T324988 | 53.124 | 0.011 | 0.859 |
| ENST00000549762 | 16.050 | 0.022 | 0.859 |
| NR_024049 | 5.36 | 0.011 | 0.859 |
| NR_040772 | 6.089 | 0.023 | 0.859 |

During real-time quantitative PCR verification stage, the expression level of ENST00000567919, ENST00000549762, NR_024049, T324988 in the MCI group is significantly higher than the normal control group, the specific data are shown in Table 4:

**Table 4: lncRNA expression levels in NC and MCI groups (2-ΔΔCt value)**

| LncRNA | NC group | MCI group | P value |
|---|---|---|---|
| NR_024049 | 0.00015465 | 0.00047339 | 0.00020 |
| ENST00000549762 | 0.00011267 | 0.00035772 | 0.00095 |
| NR_040772 | 0.00016132 | 0.00020393 | 0.30752 |
| T324988 | 0.17814497 | 0.74305331 | 0.00359 |
| ENST00000567919 | 0.00038451 | 0.00111883 | 0.00057 |

ROC curve analysis shows that four lncRNAs, NST00000567919, ENST00000549762, NR_024049, and T324988, as biomarkers have high diagnostic value for MCI.

The above four lncRNAs are independent variables, and the four lncRNAs are individually or jointly subjected to Logistic regression fitting, and a predicted probability value is calculated. The ROC curve analysis is performed again on the values obtained from the calculation model (calculation model 1, calculation model 2, calculation model 3, calculation model 4, calculation model 5, calculation model 6, calculation model 7, calculation model 8, and calculation model 9). The results show that: the numerical value of the calculation model still has high diagnostic value.
The calculation model 1 is:
   COMPUTE joint diagnosis = -4.749 +7972.194×ENST00000549762 +5609.072×NR_024049 +5.073×T324988 +961.747×ENST00000567919,
   the AUC is 0.941, the limit value is -0.8392, the sensitivity is 92%, and the specificity is 84%.
The calculation model 2 is:
   COMPUTE joint diagnosis 123 = -4.628+8552.604×ENST00000549762+5885.819× NR_024049+6.017×T324988,
   the AUC is 0.935, the limit value is -0.6984, the sensitivity is 88%, and the specificity is 84%.
The calculation model 3 is:
   COMPUTE joint diagnosis 124 = -4.549+9376.777×ENST00000549762+6908.534× NR_024049+2195.991×ENST00000567919,
   the AUC is 0.926, the limit value is -0.5859, the sensitivity is 90%, and the specificity is 84%.
The calculation model 4 is:
   COMPUTE joint diagnosis 234 = -4.220+6823.570×NR_024049+5.849×T324988+ 1213.731×ENST00000567919,
   the AUC is 0.920, the limit value is -0.3367, the sensitivity is 88%, and the specificity is 84%.
The calculation model 5 is:
   COMPUTE joint diagnosis 134 = -4.129+6.122×T324988+1128.888× ENST00000567919+9678.308×ENST00000549762,
   the AUC is 0.930, the limit value is -0.2550, the sensitivity is 90%, and the specificity is 90%.
The calculation model 6 is:
   COMPUTE Alone Diagnostic = ENST00000549762,
   the AUC is 0.846, the limit value is 0.000160542039, the sensitivity is 80%, and the specificity is 80%.
The calculation model 7 is:
   COMPUTE Alone Diagnostic = NR_024049,
The AUC is 0.877, the limit value is 0.000235892192, the sensitivity is 82%, and the specificity is 86%.
The calculation model 8 is:
   COMPUTE Alone Diagnostic = T324988,
   the AUC is 0.900, the limit value is 0.243255710000, the sensitivity is 92%, and the specificity is 82%.
The calculation model 9 is:
   COMPUTE Alone Diagnostic = ENST00000567919,
   the AUC is 0.856, the limit value is 0.000502313314, the sensitivity is 82%, and the specificity is 80%.

The analysis method indicates that the SPSS 24.0 software package is used for data analysis, and for the comparison between the two groups, a variance alignment test is first performed, and for two groups of data with the same variance, a T test is used for comparative analysis, and a P value < 0.05 is considered to have statistical significance. Binary Logistic regression is performed on the number with statistical difference to obtain a predicted probability value, and the predicted probability value is used for subsequent ROC curve analysis. The ROC curve is used to evaluate the value of lncRNA in MCI diagnosis, and the closer the curve is to 1, it indicates that the higher the diagnostic value of the indicator is.

At present, the biological diagnosis of MCI relies on expensive brain age spot PET-CT detection and invasive and difficult-to-promote lumbar puncture examination, without convenient minimally invasive peripheral plasma examination early diagnosis technology.

The beneficial effects are: through rigorous experiments and statistical analysis, the biomarkers NST00000567919, ENST00000549762, NR_024049, and T324988 with high diagnostic value for amnestic mild cognitive impairment are disclosed. Through the development and application of lncRNA markers and diagnostic kits, we have broken through the dilemma of lacking peripheral plasma diagnostic markers of MCI, which is conducive to the early diagnosis and early intervention of Alzheimer's disease dementia. At the same time, it is hopeful to greatly promote the discovery of new anti-AD therapeutic drug targets with potential therapeutic value and provide scientific basis and clinical support.

In this specification, the present invention has been described with reference to its specific embodiments. However, it is obvious that various modifications and changes can still be made without departing from the spirit and scope of the present invention. Therefore, the description and drawings should be regarded as illustrative rather than restrictive.

## Claims

1. A diagnostic marker for MCI, wherein the marker is plasma lncRNA comprising one or more of ENST00000549762, NR_024049, T324988 or ENST00000567919.

2. Application of the diagnostic marker for MCI according to claim 1 in the preparation of a diagnostic kit for MCI.

3. A diagnostic kit for MCI, wherein the kit for measuring the content of one or more of ENST00000549762, NR_024049, T324988 or ENST00000567919.

4. The diagnostic kit for MCI according to claim 3, wherein the kit comprises primers and probes of one or more of ENST00000549762, NR_024049, T324988 or ENST00000567919.

5. The diagnostic kit for MCI according to claim 3, wherein the kit comprises internal control 18S.

6. The diagnostic kit for MCI according to claim 3, wherein the formula for calculating the content of one or more of ENST00000549762, NR_024049, T324988 or ENST00000567919 in plasma measured by the kit is:
(1) -4.749+7972.194×ENST00000549762+5609.072×NR_024049+5.073×T324988 +961.747×ENST00000567919;
(2) -4.628+8552.604×ENST00000549762+5885.819×NR_024049+6.017×T324988;
(3) -4.549+9376.777×ENST00000549762+6908.534×NR_024049+2195.991 ×ENST00000567919;
(4) -4.220+6823.570×NR_024049+5.849×T324988+1213.731×ENST00000567919;
(5) -4.129+6.122×T324988+1128.888×ENST00000567919+9678.308 ×ENST00000549762;
(6) ENST00000549762;
(7) NR_024049;
(8) T324988; or,
(9) ENST00000567919.

7. A method of using the diagnostic kit for MCI according to any one of claims 3 to 6, wherein comprising:
(1) Using a lncRNA reverse transcription kit to reverse transcript the total RNA to be detected to obtain corresponding cDNA;
(2) performing real-time fluorescence quantitative PCR with the cDNA, and using 18S as an internal control, detection result is expressed as ΔCt, wherein ΔCt= Ct lncRNA - Ct 18S; and
(3) processing the result ΔCt according to a formula:
-4.749+7972.194×ENST00000549762+5609.072×NR_024049+5.073×T324988+961.747 ×ENST00000567919;
-4.628+8552.604×ENST00000549762+5885.819×NR_024049+6.017×T324988;
-4.549+9376.777×ENST00000549762+6908.534×NR_024049+2195.991 ×ENST000000567919;
-4.220+6823.570×NR_024049+5.849×T324988+1213.731×ENST000000567919;
-4.129+6.122×T324988+1128.888×ENST00000567919+9678.308×ENST000000549762; ENST000000549762;
NR_ 024049;
T324988; or,
ENST000000567919.
